# EUROPÄISCHE PATENTSCHRIFT

(11) **EP 3 784 191 B1**
(45) Veröffentlichungstag und Bekanntmachung des Hinweises auf die Patenterteilung: **12.02.2025**
(21) Anmeldenummer: 19717805.6
(22) Anmeldetag: 09.04.2019
(51) Int. Cl.: A61F 15/00, A61F 17/00, B65D 83/00, A61B 50/00, A61B 50/30, A61B 50/31

(54) **NOTHILFEMATERIALEINHEIT**
EMERGENCY AID MATERIAL UNIT
UNITÉ DE MATÉRIEL D'AIDE D'URGENCE

(30) Priorität: 23.04.2018 DE 102018109741
(43) Veröffentlichungstag der Anmeldung: 03.03.2021
(73) Patentinhaber: IVF Hartmann AG, 8212 Neuhausen am Rheinfall (CH)
(72) Erfinder: ROTHMAIER, Markus, 8308 Illnau (CH); ETTLIN, Karin, 5430 Wettingen (CH); BERNET, Claudia, 8460 Marthalen (CH); GERBER, Thomas, 3007 Bern (CH); VOGT, Rainer, 4104 Oberwil (CH)
(74) Vertreter: DREISS Patentanwälte PartG mbB
(86) Internationale Anmeldenummer: PCT/EP2019/058894
(87) Internationale Veröffentlichungsnummer: WO 2019/206615

(56) Entgegenhaltungen:
- EP-A1- 0 354 172
- WO-A2-2009/036746
- US-A- 5 718 245
- US-A1- 2005 016 230
- ANONYMOUS: "Aspire 16oz Reclosable Zipper Stand up Drink Pouches Bags w/Handle, FDA Compliant-Clear", 6 January 2017 (2017-01-06), XP055599582, Retrieved from the Internet <URL:https://www.amazon.com/Aspire-Reclosable-Zipper-Stand-up-Compliant-Clear/dp/B01N5MMTRP/ref=sr_1_98?keywords=reclosable+pouch+bags&qid=1561532437&s=gateway&sr=8-98> [retrieved on 20190626]

## Beschreibung

Die Erfindung betrifft eine Nothilfematerialeinheit umfassend mindestens ein Aufnahmekompartment zur Aufnahme von Nothilfematerial, wobei jedes Aufnahmekompartment einen geschlossenen, öffenbaren Aufnahmeraum aufweist, der zwischen zwei entlang einer geschlossen verlaufenden Umfangsnaht verbundenen Lagen ausgebildet ist.

Nothilfematerialeinheiten sind im Stand der Technik bekannt, und werden in verschiedenen Ausgestaltungen und Ausstattungen dargeboten. Die wohl bekannteste Art dürfte der Kfz-Verbandkasten sein, wobei derartige Verbandmaterialbehältnisse über DIN-Normen bezüglich ihrer in derartigen Materialeinheiten enthaltenen Gegenständen und Utensilien sowie weiterer Parameter definiert sind.

Dabei soll unter Nothilfematerialeinheit eine Zusammenstellung mehrerer Nothilfematerialien, wie Verbandspäckchen, Kompressen, Binden, Abbindemittel, etc. verstanden werden, die gemeinsam konfektioniert sind. In welcher Form oder Quantität die Zusammenstellung erfolgt, soll dabei unerheblich sein.

Beispielsweise ist aus der EP 1 714 628 A1 ein Verbandset bekannt, bei dem eine Volumenreduktion durch Komprimierung beschrieben ist.

Eine weitere Nothilfematerialeinheit ist aus der DE 10 2005 063 227 vorbekannt, die eine Anordnung beschreibt, bei der eine längliche Hülle quer in mindestens zwei Aufnahmekompartments unterteilt ist und die Aufnahmekompartments bewegbar miteinander verbunden sind.

Neben diesen eher auf den Kfz-Bereich gerichteten Nothilfematerialeinheiten sind Einrichtungen und Halterungen für Abbindeeinrichtungen bekannt, die beispielsweise bei militärischen Ausrüstungen oder auch im Bereich der zivilen Rettung mitgeführt werden. Eine solche Gestaltung ist in der DE 20 2008 008 183 U1 beschrieben, die ein Tourniquet Release System offenbart. Ein weiteres Tourniquet Release System ist beispielsweise aus der US 2014/0027480 A1 bekannt, die die Anordnung eines solchen in Holsterform zeigt, wobei die Abbindeeinheit in ein Aufnahmegehäuse, das an einer Uniform festgelegt werden kann, eingeschoben werden kann.

Schließlich beschreibt die WO 2014/182372 A1 ein Trauma Management Kit umfassend einen einfach lösbaren Beutel, um Wundverbandmaterial aufzunehmen, sowie ein Abbindeeinrichtungsholster, das auf der Außenseite des Beutels angeordnet sein kann und eine Abbindeeinrichtung aufnimmt, sowie eine Abdeckung für das Holster, die an dem Beutel festgelegt ist. Mit einem derartigen Trauma Management Kit soll insbesondere bei einer Verletzung mit massivem Blutverlust eine erste Wundstillung bereitgestellt werden können, wobei es wichtig ist, dass der Zugang zur Abbindeeinheit noch vor dem Zugang zu den Verbandmitteln ermöglicht ist, da dies über Leben und Tod entscheiden kann.

Ferner beschreibt die EP 0354 172 A1 eine Verpackung von saugfähigen Gegenständen, wie Windeln, mit mehreren Aufnahmefächern für die Windeln.

Die WO 2009/036746 A2 beschreibt eine Vorrichtung zum Bevorraten und/oder Bereithalten von Nothilfematerialien mit verschieden ausgebildeten Aufnahmekompartments.

Aus der US 5,718,245 B ist eine Einmalverpackung für Nothilfematerial mit einem gemeinsamen Aufnahmekompartment bekannt.

Der vorliegenden Erfindung stellt sich nun die Aufgabe, eine Nothilfematerialeinheit bereitzustellen, die eine einfache Zugänglichkeit zu den darin beinhalteten Nothilfematerialien ermöglicht und insbesondere vielfältige Einsatzmöglichkeiten und Anbringungsmöglichkeiten bereitstellt.

Dabei sollen die Nothilfematerialien insbesondere zur Erstversorgung von Verletzungen dienen, wie sie insbesondere bei Traumata auftreten, die häufig mit stark blutenden Wunden einhergehen. Für solche Einsatzzwecke, die auch als Krisensituationen beschrieben werden können, ist es notwendig, einen schnellen Zugriff zu Verbandsmaterialien und insbesondere auch zu Abbindemitteln zu haben. Gerade bei stark blutenden Wunden ist es wichtig, einen hinreichenden Druck aufzubringen, so dass es besonders wünschenswert ist, eine entsprechende Einrichtung, die dies auch für Laien und weniger kräftige Personen ermöglicht, bereitzustellen.

Neben der bekannten Bereitstellung für insbesondere in Krisensituationen tätige Personen, wie Feuerwehrleute, technische Hilfswerke, Sanitäter oder Soldaten, besteht auch der Wunsch, solche Nothilfematerialeinheiten im öffentlichen Raum anzuordnen. Die Anordnung von Nothilfematerialien, wenn auch mit anderer Zielrichtung, im öffentlichen Raum hat sich zwischenzeitlich in Form von Defibrillatoren, die in vielen öffentlichen Gebäuden angeordnet sind, in gewisser Weise etabliert.

Die Erfindung löst diese Aufgabe durch eine Nothilfematerialeinheit gemäß Anspruch 1, wobei mindestens zwei Aufnahmekompartmente vorgesehen sind und jedes Aufnahmekompartment mindestens ein Griffelement aufweist, wobei das mindestens eine Griffelement bügelförmig ausgebildet ist. Durch ein derartiges Griffelement wird erreicht, dass eine Nothilfematerialeinheit, die in einem Holster oder einer anderen Halterung aufgenommen ist, immer auf den ersten Griff sicher am Griffelement ergriffen werden kann und so schneller im Notfall zur Verfügung steht.

Besonders bevorzugt ist es dabei, dass mindestens eine, vorzugsweise beide Lagen, des Aufnahmekompartments mit einem Griffelement verbunden sind. Auf diese Weise ist eine besonders einfache Ausgestaltung des Griffelements möglich, nämlich, wenn das Griffelement integral mit der jeweiligen Lage, bei der es sich vorzugsweise um eine Folienschicht aus einem thermoplastischen Material, bevorzugt Polyethylen, Polypropylen, Polyamid oder Polyethylenterephthalat, einem Siegelpapier oder einer Siegelfolie handeln kann, verbunden ist. Das Griffelement kann dabei durch einen Bereich von einer oder beiden Lagen gebildet werden, der außerhalb der Umfangsnaht angeordnet ist.

Dabei kann die Umfangsnaht beispielsweise eine thermische oder Ultraschallschweißnaht sein, die die beiden Lagen, die insbesondere als Folien ausgebildet sind, miteinander fügt, wobei die Fügung vorzugsweise so verläuft, dass der Aufnahmeraum vollumfänglich verschlossen ist, insbesondere auch flüssigkeitsdicht und insbesondere auch gas- oder bakteriendicht verschlossen ist.

Es ist dabei vorgesehen, dass das mindestens eine Griffelement bügelförmig ausgebildet ist. Eine bügelförmige Ausbildung kann dadurch erfolgen, dass sich die Lagen nach außerhalb der Umfangsnaht erstrecken und dann eine Ausstanzung vorgenommen wird, so dass ein bügelförmiges Element der Lagen stehen bleibt.

Besonders bevorzugt und einfach ist dabei auch eine Verpackung, bei der das Aufnahmekompartment statt aus zwei Folienlagen als Blisterverpackung ausgebildet ist und eine der Lagen als Schalenelement und die andere Lage als Deckelelement ausgebildet ist. Dabei wird das Schalenelement im Wesentlichen dreidimensional ausgebildet, wohingegen das Deckelelement im Wesentlichen flach ist und den Aufnahmeraum, der durch das Schalenelement gebildet wird, verschließt.

In diesem Fall kann z.B. nur das Deckelement mit dem Griffelement verbunden sein.

Es ist vorgesehen, dass mindestens zwei Aufnahmekompartments vorgesehen sind, die insbesondere miteinander verbunden sind. Durch die Vorsehung von mehr als einem Aufnahmekompartment kann das Nothilfematerial übersichtlicher und besser strukturiert dargeboten werden.

Dabei ist es vorteilhaft vorgesehen, wenn sich die Griffelemente flächig überdecken, wobei dies sowohl gilt, sofern mehr als ein Griffelement bzw. ein aus zwei Lagen bestehendes Griffelement je Aufnahmekompartment vorgesehen sind, als auch wenn mehrere Aufnahmekompartments vorgesehen sind, die dann jeweils ein oder mehrere Griffelemente bzw. Griffelementlagen aufweisen. Dabei ist es besonders vorteilhaft, wenn die Aufnahmekompartments und/oder die Griffelemente in Richtung senkrecht zur Flächenrichtung übereinander anordenbar sind und sich in dieser Anordnung flächenmäßig überdecken. Hierdurch kann eine besonders kompakte Anordnung geschaffen werden, in der das benötigte Nothilfematerial auf besonders kleinem Raum und optisch entsprechend dargeboten werden kann.

Eine besonders einfache Gestaltung wird dadurch geschaffen, dass die Aufnahmekompartments untereinander über ein Filmscharnier an einer vorzugsweise nicht das Griffelement aufweisenden Seitenkante verbunden sind, wobei insbesondere entlang des Filmscharniers Sollbruchstellen, insbesondere in Form von Perforationen, vorgesehen sein können. Durch eine derartige Gestaltung ist eine besonders einfache Herstellbarkeit der Aufnahmekompartments möglich. Insbesondere können diese durch eine oder zwei durchgehende Lagen gebildet oder verbunden sein und das Filmscharnier kann durch die eine Umfangsnaht ausgebildet sein, die das Aufnahmekompartment verschließt und gleichzeitig ein Filmscharnier ausbildet. Alternativ kann das Filmscharnier auch durch einen Bereich zwischen den Umfangsnähten ausgebildet sein.

Sofern gewünscht, dass die Aufnahmekompartments einfach voneinander trennbar sind, insbesondere ohne die Aufnahmeräume zu verletzen, kann vorgesehen sein, dass entlang des Filmscharniers Sollbruchstellen, insbesondere in Form von Perforationen, vorgesehen sind.

Statt der Verbindung der Aufnahmekompartments über Filmscharniere kann auch vorgesehen sein, dass die Aufnahmekompartments über magnetische, komplementäre Elemente miteinander lösbar verbindbar sind. Dies ermöglicht eine noch einfachere Separierung voneinander und verringert die Gefahr, dass ein Aufnahmekompartment ungewollt beim Versuch des Trennens der Aufnahmekompartments voneinander aufgerissen und somit zerstört wird.

Darüber hinaus kann vorgesehen sein, dass zwischen den Aufnahmekompartments ein Erweiterungskompartment vorgesehen ist bzw. dazwischen eingebracht werden kann, das ebenfalls einen Aufnahmeraum aufweist, jedoch keine Griffelemente umfasst. Dieses Erweiterungsmodul kann insbesondere auch über ein Filmscharnier und/oder magnetische komplementäre Elemente mit den Aufnahmekompartments verbunden sein.

Besonders bevorzugt ist darüber hinaus, dass mindestens eines der Aufnahmekompartments, vorzugsweise jedes der Aufnahmekompartments, eine Öffnungshilfe aufweist. Hierdurch wird die Öffnung des öffenbaren Aufnahmekompartments erleichtert und insbesondere kann auch erreicht werden, dass eine definierte Öffnungsweise erreicht wird und das Kompartment nicht in beliebiger Weise geöffnet wird bzw. schlicht zerrissen wird, wodurch Nothilfematerialien aus dem Aufnahmekompartment herausfallen können, wenn die Öffnung nicht wie gewünscht erfolgt.

Besonders bevorzugt ist es dabei, wenn als Öffnungshilfe das Griffelement dient. Auf diese Weise können zwei Funktionen im Griffelement integriert sein.

Weiterhin ist es besonders bevorzugt, wenn die Nothilfematerialeinheit eine Befestigungsöffnung aufweist. Diese kann insbesondere bezüglich des Aufnahmeraums dem Griffelement gegenüberliegend angeordnet sein und außerhalb des Aufnahmeraums liegen, zum Befestigen an einer Halteeinrichtung. Als derartige Halteeinrichtungen kommen sowohl Holster als auch Abgabeeinrichtungen, Dispenser, aber auch Gürtel, Uniformelemente, Schlüsselbänder oder ähnliches in Frage, an denen eine entsprechende Nothilfematerialeinheit angebracht werden kann. Alternativ sind auch andere Befestigungsmöglichkeiten denkbar, z.B. auch über das Griffelement. Weiterhin ist eine Befestigungsmöglichkeit denkbar, in der das Aufnahmekompartment über eine magnetische oder eine andere kraft- oder formschlüssige oder materialschlüssige Verbindung befestigt ist. Die Befestigungsöffnung stellt jedoch eine vergleichsweise einfache Möglichkeit dar. Insbesondere, wenn es sich um ein zerstörbares Material handelt, wobei gegebenenfalls entsprechende Schwächungen und Sollbruchstellen vorgesehen sein können, ist eine rasche Entnahme und Bereitstellung der Nothilfematerialeinheit dadurch möglich, dass diese nicht erst mühsam von einer Halteeinrichtung entfernt werden muss, sondern einfach unter Zerstörung eines die Befestigungsöffnung einschließenden Materialbereichs von der Halteeinrichtung abgerissen werden kann.

Die Erfindung umfasst ferner einen Nothilfematerialeinheitdispenser aufweisend eine Nothilfematerialeinheit der beschriebenen Art, wobei der Dispenser ein Gehäuse zur Aufnahme der Nothilfematerialeinheit und eine Gehäuseöffnung aufweist, durch die die Nothilfematerialeinheit entnommen werden kann mit den Merkmalen des Anspruchs 15.

Dabei ist es besonders bevorzugt, wenn Gehäuse und Gehäuseöffnung so ausgebildet sind, dass sich mindestens das eine Griffelement bei in das Gehäuse aufgenommener Nothilfematerialeinheit aus der Öffnung heraus erstreckt. Dadurch ist eine besonders einfache Entnehmbarkeit gewährleistet. Der Rest der Nothilfematerialeinheit, der nicht aus der Öffnung herausragt, bleibt durch den Nothilfematerialeinheitdispenser gut geschützt.

Besonders bevorzugt ist es dabei, wenn das Gehäuse eine Rückwand und eine Abdeckung aufweist, die zur Bildung des Gehäuses lösbar miteinander verbindbar sind. Hierüber sind eine einfache Anbringbarkeit beispielsweise an einer Wand und eine einfache Wechselbarkeit der Nothilfematerialeinheit möglich. Insbesondere kann vorgesehen sein, dass der Nothilfematerialeinheitdispenser eine Halte- oder Trageeinrichtung aufweist, die eine Befestigungsöffnung in der Nothilfematerialeinheit bei aufgenommener Nothilfematerialeinheit durchgreift.

In einer bevorzugten Ausführungsform weist der Nothilfematerialeinheitdispenser ein zusätzliches Schutzelement auf, welches wenigstens ein Griffelement überdeckt. Ein derartiges Schutzelement erhöht den Aufwand zur Entnahme der Nothilfematerialeinheit ohne sie zu verhindern und erschwert damit eine missbräuchliche Entnahme der Nothilfematerialeinheit. Dieses Schutzelement ist mit dem Gehäuse verbunden und muss vor der Entnahme der Nothilfematerialeinheit bewegt, z.B. verschoben oder verschwenkt oder entfernt werden, so dass das Griffelement freigelegt wird. Dabei kann dieses Element vollständig entfernt werden oder mit einem Teil des Gehäuses unlösbar verbunden bleiben, beispielsweise über eine scharnierartige Verbindung. Bevorzugt ist eine Ausgestaltung, bei der das Schutzelement scharnierartig mit der Rückwand des Gehäuses verbunden ist, und gleichzeitig lösbar mit der Abdeckung des Gehäuses verbunden ist, so dass in einem ersten Zustand das Schutzelement die gesamte oder einen Teil der Gehäuseöffnung überdeckt und insbesondere bündig mit den Außenkanten von Rückwand und Abdeckung abschließt. Das Schutzelement wirkt daher als öffenbares Abdeckungsmittel für die Gehäuseöffnung. D.h. das Schutzelement verschließt oder verkleinert die Gehäuseöffnung.

Die lösbare Verbindung des Schutzelements mit dem Gehäuse kann kraftschlüssig, formschlüssig oder stoffschlüssig erfolgen. Das Schutzelement kann aus einem beliebigen Material gefertigt sein, bevorzugt jedoch aus einem transparenten Kunststoff, beispielsweise Polyethylen, Polypropylen, Polyamid, Polycarbonat, Polyester, Polyethylenterephthalat. Dieses Schutzelement kann mit einer zusätzlichen Vorrichtung versehen sein, die die Entfernung des Schutzelements erleichtert, beispielsweise eine Grifföffnung oder Griffmulde oder ein Griffelement in Form eines vorstehenden Materialabschnitts oder Henkels.

Grundsätzlich ist es auch denkbar, dass die Entfernung des Schutzelements nicht wie beschrieben wiederholt möglich ist, sondern dass das Schutzelement bei der Entfernung zerstört werden muss. Es kann dabei vorgesehen sein, dass hierzu Hilfsmittel vorgesehen sind.

Darüber hinaus kann der Nothilfematerialeinheitdispenser weitere Elemente aufweisen, wie insbesondere eine optische oder akustische Signaleinrichtung, eine Kameraeinrichtung, mit der Videos oder Bilder vom Unfallort erstellt werden können, und/oder eine Codierung. Darüber hinaus kann auch eine Kommunikationseinrichtung vorgesehen sein. Als Nothilfematerialien können beispielsweise Einweghandschuhe, Verbandmaterialien, aber auch eine Patiententransferunterlage, eine Rettungsdecke und/oder ein Thoraxpflaster mit Ventil, sowie ein Beatmungstubus und/oder ein Mundschutz vorgesehen sein. Weitere mögliche Nothilfematerialien sind eine Abbindeeinrichtung, eine Blutverlustschablone, mittels derer beispielsweise der Laie eine Analogie zum Blutverlust geben kann, so dass wichtige und wertvolle Informationen für die Aufnahme in ein Krankenhaus gegeben werden. Ferner können Patientenmarkierungen, beispielsweise in Form von Flaggen, vorgesehen sein, ähnlich wie bei der Lawinenrettung. Hierüber ist gegebenenfalls auch eine Markierung und Priorisierung über den Schweregrad oder die Art der Verletzung möglich. Wie bereits ausgeführt, kann die Nothilfematerialeinheit in einem

Nothilfematerialeinheitdispenser untergebracht sein, und so insbesondere im öffentlichen Raum, wie beispielsweise in Banken, Sporteinrichtungen, Schulen, etc. vorgesehen und sichtbar für den Bedarfsfall dargeboten werden. Eine Benutzung ist darüber hinaus auch ohne Nothilfematerialeinheitdispenser möglich. Beispielsweise als Teil einer Ausrüstung von zivilen Personen und/oder der Polizei bzw. Rettungskräften. In diesem Fall kann eine Halterung über die Griffelemente und/oder über eine, sofern vorgesehen, Befestigungsöffnung erfolgen. Darüber hinaus lässt sich eine derartige Nothilfematerialeinheit gegebenenfalls auch mittels Drohnen an einen Bedarfsort bringen.

Sofern das Notfhilfeverbandset mehr als ein Aufnahmekompartment aufweist, ist es besonders vorteilhaft, wenn jedes Aufnahmekompartment mit verschiedenen Nothilfematerialien bestückt ist. So kann beispielsweise das eine Aufnahmekompartment eine Abbindeeinrichtung und das andere Kompartment weitere Maßnahmen zur Blutstillung und Wundversorgung aufweisen.

Darüber hinaus kann der Nothilfematerialeinheitdispenser auch weitere Hilfsmittel für den Nothilfefall, wie optische oder akustische Signal- oder Sendeeinrichtung aufweisen. Ferner kann ein Code, z.B. ein QR-Code vorgesehen sein, dem Informationen, z.B. Standort, entnommen werden können. Schließlich sind auch Kameraeinrichtungen bekannt. Der Nothilfematerialeinheitdispenser kann zur besseren Auffindbarkeit Markierungen, z.B. in Form des bekannten "Kreuzes" aufweisen.

Nach einer weiteren alternativen Ausgestaltung ist es auch möglich, eine Vielzahl von Aufnahmekompartments vorzusehen, die in einem Nothilfematerialeinheitdispenser untergebracht sind, wobei der Nothilfematerialeinheitdispenser hier vorzugsweise so ausgestaltet ist, dass er als Ganzes vom Aufbewahrungsort gelöst werden kann und mittels Tragevorrichtung zum Bedarfsort gebracht wird. ++++++++ Weitere Vorteile und Nachteile ergeben sich aus der nachfolgenden Figurenbeschreibung sowie der übrigen Beschreibung und den Ansprüchen. Sämtliche Merkmale sind dabei sowohl für sich alleine als auch in Kombination erfindungswesentlich.

Dabei zeigen:
Figur 1 eine erste Ausgestaltung eines Dispensers mit Nothilfematerialeinheit;
Figur 2 eine erste Ansicht der Nothilfematerialeinheit;
Figur 3
   A bis F eine Verwendung der Nothilfematerialeinheit;
Figur 4
   A bis C verschiedene Varianten der Nothilfematerialeinheit;

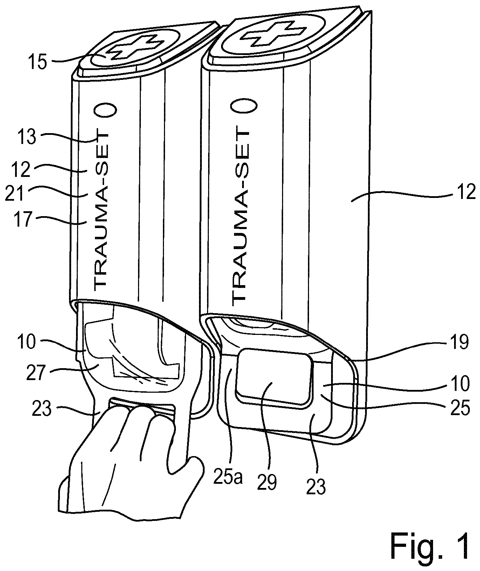

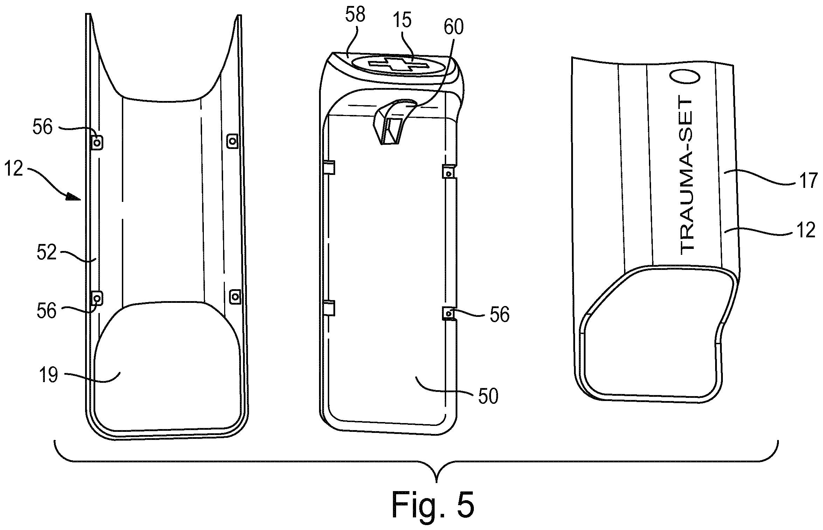

Figur 5 den Dispenser gemäß Figur 1 in einer geöffneten Darstellung;

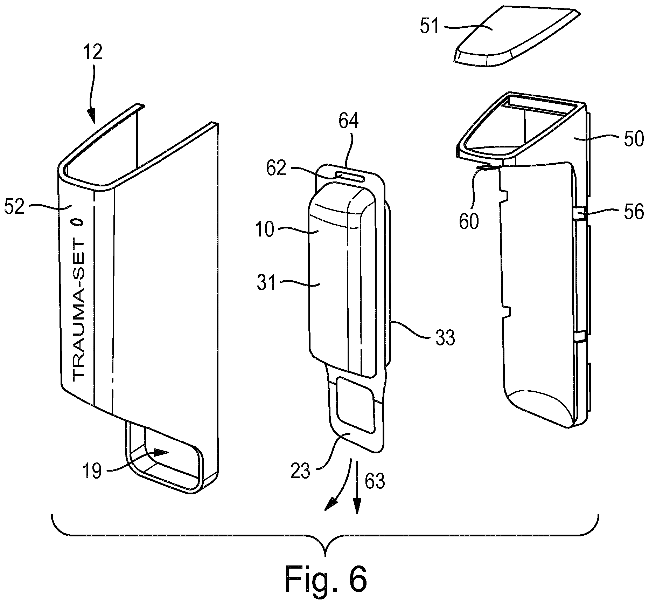

Figur 6 eine Explosionsdarstellung des Dispensers mit Nothilfematerialeinheit;
Figur 7
   A und B eine alternative Gestaltung eines Dispensers mit einem zusätzlichem Schutzelement als Diebstahlsicherung und
Figur 8
   A und B eine alternative Gestaltung eines Dispensers.

Figur 1 zeigt eine Nothilfematerialeinheit 10, die in einem Dispenser 12 aufgenommen ist. In der vorliegenden Ausgestaltung gemäß Figur 1 sind hierbei unmittelbar nebeneinander zwei Dispenser mit jeweils einer

Nothilfematerialeinheit 10 vorgesehen. Der Dispenser ist durch Aufbringen eines Schriftzugs (Markierung) "Traumaset" oder ähnliches, der mit dem Bezugszeichen 13 versehen ist, gekennzeichnet, um auf den Inhalt aufmerksam zu machen. Darüber hinaus ist auf dem Dispenser das international übliche Zeichen des Kreuzes angebracht (Bezugszeichen 15), das auf Materialien zur Nothilfe hinweist.

In jedem der Dispenser 12 ist eine Nothilfematerialeinheit 10 vorgesehen. Der Dispenser 12 umfasst dabei ein Gehäuse 17 und eine Gehäuseöffnung 19, wobei die Gehäuseöffnung 19 u.a. dadurch gebildet wird, dass die Vorderseite 21, die bei Gebrauch dem Benutzer zugewandt ist, in Längsrichtung des Dispensers 12 verkürzt ausgebildet ist, so dass ein Griffelement 23 der Nothilfematerialeinheit 10 aus dem Gehäuse 17 in der figürlichen Darstellung nach unten herausragt. Das Griffelement 23 ist dabei bügelartig 25 ausgebildet und kann insbesondere farbig gekennzeichnet oder auch verstärkt ausgebildet sein, um eine leichte Erkennbarkeit zu gewährleisten und die Funktion deutlich zu machen und zu erkennen.

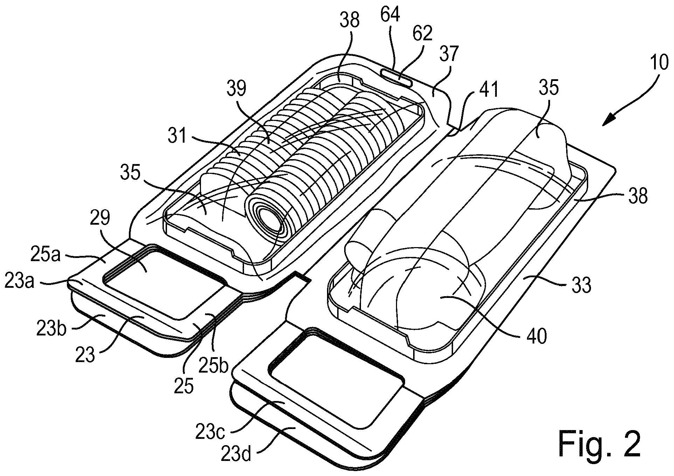

Das bügelartige Griffelement 23 ist so ausgebildet, dass die beiden Seitenteile 25a und 25b jeweils an einem Aufnahmekompartment 27 der Nothilfematerialeinheit 10 angelenkt sind und sich so zwischen dem Griffelement 23 und dem Aufnahmekompartment 27 eine Öffnung 29 ausbildet, in die die Finger einer Hand, wie bei dem linken Dispenser 12 in der Darstellung gezeigt, eingreifen können und in der vorliegenden Anordnung die Nothilfematerialeinheit 10 nach unten aus dem Dispenser 12 herausziehen können. Die Nothilfematerialeinheit 10 umfasst dabei in Figur 1 zwei Folienabschnitte, die übereinandergelegt durch Miteinanderverschweißen zwei Aufnahmeräume 35 zweier Aufnahmekompartments 31, 33 ausbilden, wobei die Aufnahmekompartments im vorliegenden Fall über Filmscharniere verbunden sind. Die Nothilfematerialeinheit 10 ist dabei in Figur 2 in aus dem Dispenser herausgenommenen Zustand gezeigt, wobei hier zwei Aufnahmekompartments 31 und 33 vorgesehen sind, die jeweils durch die Bildung eines Aufnahmeraums 35 für das Nothilfematerial gestaltet sind, wobei der Aufnahmeraum 35 durch eine Umfangsnaht, die durch thermisches oder Ultraschallverschweißen oder weitere bekannte Maßnahmen allseitig abgeschlossen ist und das Nothilfematerial in sich einschließt. Die Umfangsnaht ist mit dem Bezugszeichen 37 versehen. Die Aufnahmekompartments 31 und 33 sind dabei als Folienverpackung gestaltet und können gegebenenfalls evakuiert und sterilisiert werden. Ebenfalls durch die Folienlagen werden die Griffelemente 23 gebildet, wobei im Bereich der Griffelemente 23 Verstärkungselemente aufgebracht sein können und zur Bildung einer Grifföffnung 29 Bereiche der Folienlagen ausgestanzt werden können.

Jedes Griffelement 23 besteht dabei aus zwei Lagen 23a und 23b, die flächig übereinander angeordnet sind und so das Griffelement 23 ergeben. Aufgrund der Vorsehung von zwei Aufnahmekompartments 31, 33 resultiert dies in insgesamt vier Grifflagen 23a, b, c, d, die in dem in den Dispenser 12 aufgenommenen Zustand der Nothilfematerialeinheit 10 allesamt übereinander angeordnet sind und ein gemeinsames Griffelement 23 bilden. Hierdurch besitzt das Griffelement 23 eine hinreichende Robustheit, um ein Entnehmen der Nothilfematerialeinheit 10 aus dem Dispenser 12 zu ermöglichen, ohne dass auch bei Anwendung einer gewissen Kraft die Nothilfematerialeinheit 10 in ungewollter Weise zerstört wird.

In die Aufnahmeräume 35 sind jeweils Kunststoffschalen 38 aufgenommen, in denen das Nothilfematerial dargeboten und angerichtet ist. Im vorliegenden Fall ist in einem der Aufnahmekompartments 31 Bindenmaterial 39 zum Anlegen eines Druckverbandes vorgesehen und in dem zweiten Aufnahmekompartment 33 eine Abbindeeinrichtung 40. Die in den Aufnahmekompartments 31 und 33 vorgesehenen Materialien können durch Aufbringung eines Schriftzugs entsprechend gekennzeichnet sein, so dass ein Benutzer sofort weiß, was in welchem Kompartment 31, 33 vorgesehen ist. Durch die Umfangsnaht 37, die die Aufnahmeräume 35 umschließt, bildet sich zwischen den Aufnahmekompartments 31 und 33 ein Filmscharnier 41 aus, das eine gelenkige Verbindung der beiden Aufnahmekompartments 31, 33 ermöglicht, so dass die beiden Aufnahmekompartments 31 und 33 im in den Dispenser 12 aufgenommenen Zustand aufeinandergelegt werden können mit der Rückseite ihrer Kunststoffschalen 38, so dass sich die vier Grifflagen 23a, 23b, 23c und 23d zu einem einzigen Griffelement 23 ergänzen.

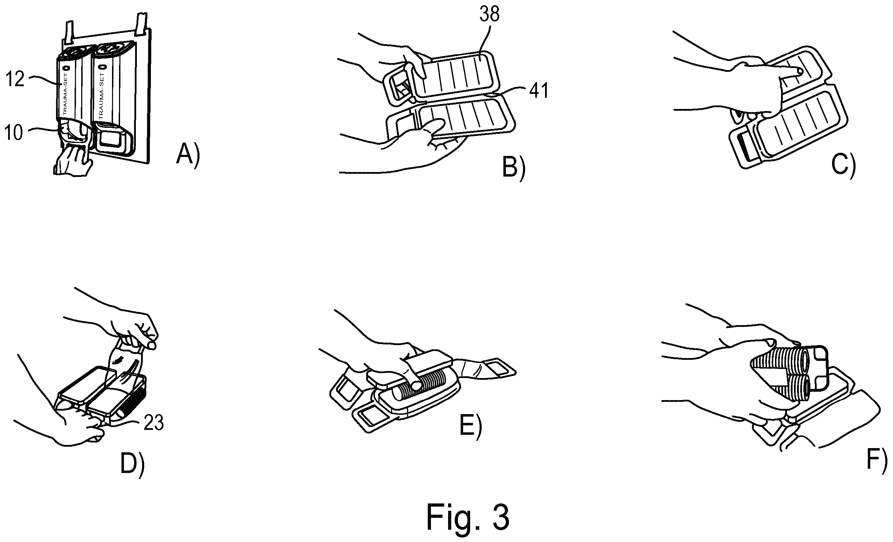

Figur 3 zeigt den Anwendungsfall einer erfindungsgemäßen Nothilfematerialeinheit 10. Figur 3 zeigt in den Abbildungen A), B), C), D), E) bis F). Abbildung A) die Entnahme der Nothilfematerialeinheit 10 aus dem Dispenser 12 gemäß Figur 1.

Die Nothilfematerialeinheiten 10 sind dabei im Dispenser 12 so gehalten, dass diese gegen versehentliches Herausnehmen oder Herausfallen gesichert sind, gleichwohl jedoch ein einfaches Entnehmen möglich ist.

Im nächsten Schritt erfolgt dann eine Verschwenkung um das Filmscharnier 41 in einer Weise, dass die Rückseiten der Kunststoffschalen 38 in Richtung des Benutzers weisen. Auf den Rückseiten der Kunststoffschalen 38 sind Gebrauchsanleitungen vorgesehen. Diese können z.B. auf das Folienmaterial oder, sofern vorgesehen, die Kunststoffschalen aufgedruckt sein oder auch lose beiliegen, was in den Figuren 3B und C gezeigt ist. Zum Herausnehmen der Nothilfematerialien aus den Aufnahmeräumen 35 werden nun die Aufnahmekompartments 31, 33 geöffnet, indem eine der Lagen 23a eines Griffelements 23 festgehalten wird und die andere Lage in der Art einer Peelbewegung in die entgegengesetzte Richtung gezogen wird, so dass die Umfangsnaht 37 entweder zerstört wird oder das Folienmaterial innerhalb der Umfangsnaht zerstört wird, so dass das in dem Aufnahmeraum 35 vorgesehen Nothilfematerial, wie in Figur 3E) gezeigt, entnommen werden kann. Das Griffelement 23 dient hier als Öffnungshilfe. Das Nothilfematerial steht dann zur Nothilfeleistung zur Verfügung.

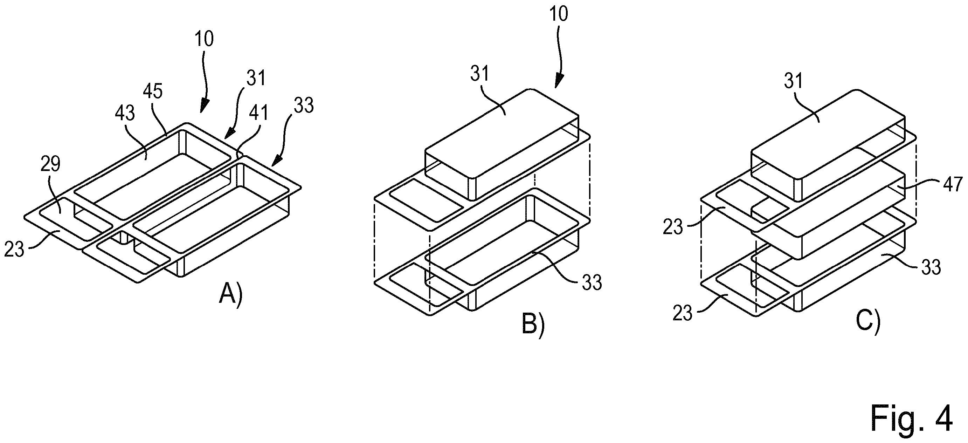

Figur 4 zeigt eine Gestaltung, bei der in Figur 4A) eine Nothilfematerialeinheit 10, wie sie in Figur 2 gezeigt ist, ähnlich ist. Die Nothilfematerialeinheit 10 ist hier jedoch in Form von zwei Blisterpackungen vorgesehen, wobei ein Schalenelement 43 je Aufnahmekompartment 31 und 33 vorgesehen ist und das Schalenelement durch ein Deckelelement 45 überdeckt und verschlossen wird. Im vorliegenden Fall ist das Griffelement 23 nicht doppellagig ausgebildet. Das Deckelelement 45 ist dabei so ausgebildet, dass es sich über beide Schalenelemente 43 erstreckt und die Griffelemente 23 ausbildet, indem das Deckelelement 45 sich über das Schalenelement 43 hinübererstreckt, in einer Form, dass durch Anbringen einer Ausstanzung 29 jeweils ein bügelartiges Griffelement 23 gebildet wird. Im Bereich zwischen den beiden Aufnahmekompartments 31 und 33 ist dann wiederum ein Filmscharnier 41 vorgesehen, das eine Klappbarkeit der beiden Aufnahmekompartments aufeinander ermöglicht.

Figur 4B zeigt nun eine alternative Gestaltung, bei der die beiden Blisterverpackungen, die die Aufnahmekompartments 31, 33 bilden, nicht über ein Filmscharnier integral miteinander verbunden sind, sondern voneinander separiert sind und in anderer Weise beispielsweise über Magnete, oder auch alternativ über Klebe- oder Schweißpunkte, aufeinander fixiert sind, wobei die Fixierung so erfolgen sollte, dass sie ohne großen Kraftaufwand nach Entnahme der Nothilfematerialeinheit 10 aus einem Dispenser 12 aufgelöst werden kann. Darüber hinaus sollte der Zusammenhalt so sicher sein, dass, sofern eine Verwendung mit einem

Dispenser 12 vorgesehen ist, die beiden Aufnahmekompartments 31 und 33 sich nicht ungewollt voneinander trennen.

Figur 4C zeigt schließlich eine Gestaltung, bei der zwischen den Aufnahmekompartments 31 und 33, die wiederum mit Magneten zusammengehalten werden, noch ein Erweiterungskompartment 47 vorgesehen ist, das zwischen den Deckelelementen 45 der Aufnahmekompartments 31, 33 zu liegen kommt, so dass die Griffelemente 23 der beiden Aufnahmekompartments 31 und 33 um die Dicke des Erweiterungskompartments 47 voneinander beabstandet sind.

Figur 5 zeigt nun den Dispenser 12 zum einen rechts in der Zeichnungsdarstellung im zusammengebauten und montierten Zustand, so wie links in der Zeichnungsdarstellung im geöffneten Zustand. Der Dispenser umfasst dabei eine Rückwand 50 sowie eine Abdeckung 52, die gemeinsam das Dispensergehäuse 17 ausbilden. Die Verbindung zwischen Rückwand 50 und Abdeckung 52 erfolgt dabei über Rastelemente 56, die korrespondierend sowohl in der Abdeckung 52 als auch in der Rückwand 52 vorgesehen sind. Die Rückwand 50 trägt im vorliegenden Fall auch ein Deckelelement 58 auf der das zuvor beschriebene Kreuz-Symbol 15 zur Kennzeichnung der Nothilfematerialeinheit 10 angeordnet ist. Die Rückwand 50 ist im Bereich der Gehäuseöffnung 19 im zusammengebauten Zustand sichtbar. Abgewandt von der Gehäuseöffnung 19 an der Rückwand 50 ist eine Halteeinrichtung 60 vorgesehen, die eine Befestigungsöffnung 62, die in Figur 2 gezeigt ist, in der Nothilfematerialeinheit durchgreifen kann. Die Befestigungsöffnung 62 ist dabei vorzugsweise an der dem Griffelement 23 abgewandten Seite der Nothilfematerialeinheit vorgesehen. Das Entnehmen der Nothilfematerialeinheit 10 aus dem Dispenser 12 erfolgt dabei unter Zerstörung einer Materialbrücke 64 (s. Figur 2), die beim nach unten Herausziehen der Nothilfematerialeinheit zerreißt. Hierzu können insbesondere entsprechende Materialschwächungen vorgesehen sein, um die gewünschte Entnahmekraft einzustellen.

Auf diese Weise kann eine sichere Halterung gegen unbeabsichtigtes Herausfallen bei gleichzeitig einfacher Gestaltung realisiert werden. Zum Befüllen des Dispensers 12 wird dabei das Gehäuse in seine Bestandteile Rückwand 50 und Abdeckung 52 getrennt, so dass dann über das Halteelement 60 die Befestigungsöffnung 62 der Nothilfematerialeinheit 10 aufgehängt werden kann und dann die Abdeckung wiederum auf der Rückwand 50 angebracht wird, so dass dann, wie in Figur 1 gezeigt, das Griffelement 23 der Nothilfematerialeinheit 10 im Bereich der Gehäuseöffnung 19 sichtbar ist.

Weitere Möglichkeiten des Mitsichführens der Nothilfematerialeinheit sind dabei über Tragen am Griffelement 23, aber auch Festlegen mittels eines Schlüsselbandes oder einer Gürteltrageeinrichtung, die entweder in das Griffelement 23 oder die Befestigungsöffnung 62 eingreifen können. Darüber hinaus ist über das Griffelement 23 sowie die Befestigungsöffnung 62 auch ein Transport beispielsweise mittels Drohnen möglich.

Figur 6 zeigt eine Gestaltung, bei der der Dispenser mit der darin angeordneten Nothilfematerialeinheit in einer Explosionsdarstellung gezeigt ist. Der Dispenser 12 besteht dabei aus der Abdeckung 52 sowie der Rückwand 50, wobei ein Deckelelement 51 als Teil der Rückwand vorgesehen ist. Abdeckung 52 und Rückwand 50 werden über die Rastelemente 56, die sowohl an der Abdeckung (Figur 5) als auch an der Rückwand 50 vorgesehen sind, miteinander lösbar rastend verbunden. Die Nothilfematerialeinheit 10 besteht dabei aus den zwei Aufnahmekompartments 31 und 33 und weist das aus der Öffnung 19 des Dispensers hinausragende Griffelement 23 auf. Zur Befestigung am Dispenser ist eine Befestigungsöffnung 62 vorgesehen, die ein entsprechendes Halteelement 60 übergreift, wobei eine Materialbrücke 64 bei Herausziehen der Nothilfematerialeinheit 10 in Pfeilrichtung 63 zerstört wird, so dass die Nothilfematerialeinheit 10 aus dem Dispenser 12 entnommen werden kann.

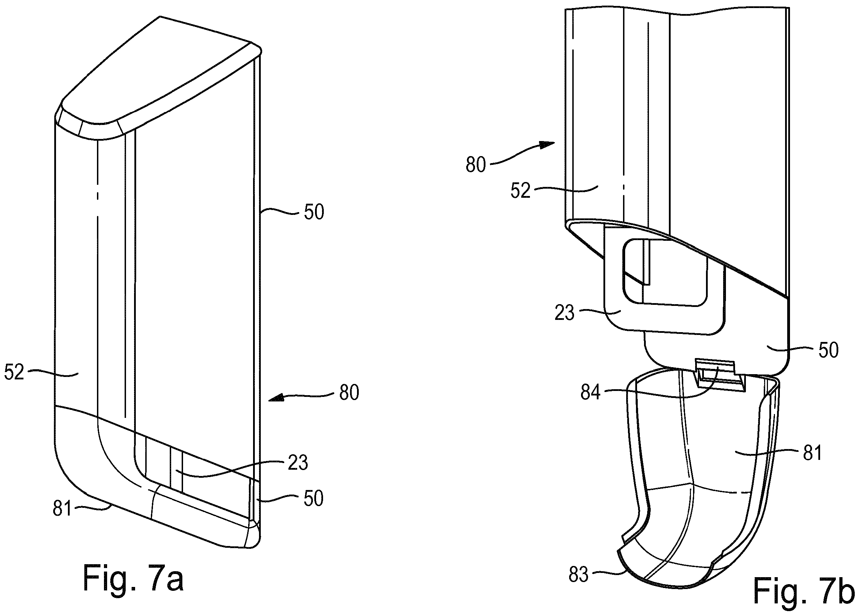

Figur 7A zeigt eine alternative Gestaltung eines Dispensers 80 mit einem zusätzlichen Schutzelement 81 in geschlossenem Zustand. Das Schutzelement 81 ist über eine scharnierartige Verbindung 84 mit der Gehäuserückwand 50 verschwenkbar verbunden. Dabei überdeckt das Schutzelement das Griffelement 23 in der Art eines Abdeckungsmittels. Das Schutzelement 81 schließt bündig mit den Außenkanten von Gehäuserückwand 50 und Gehäuseabdeckung 52 ab und ergänzt somit das Gehäuse zu einer geschlossenen Gestaltung.

Figur 7B zeigt dieselbe alternative Gestaltung eines Dispensers 80 mit einem zusätzlichen Schutzelement 81 in geöffneten Zustand. Das Schutzelement 81 weist an seiner mit der Gehäuseabdeckung 52 abschließenden Vorderkante 82 ein Verschlusselement 83 auf, welches so ausgestaltet ist, dass es auf der Innenseite der vorderen Kante 84 der Gehäuseabdeckung 52 freigelegt werden kann, z.B. dort kraft- oder formschlüssig hält. Das Schutzelement 81 ist über eine scharnierartige Verbindung 84 mit der Gehäuserückwand 50 unlösbar verbunden.

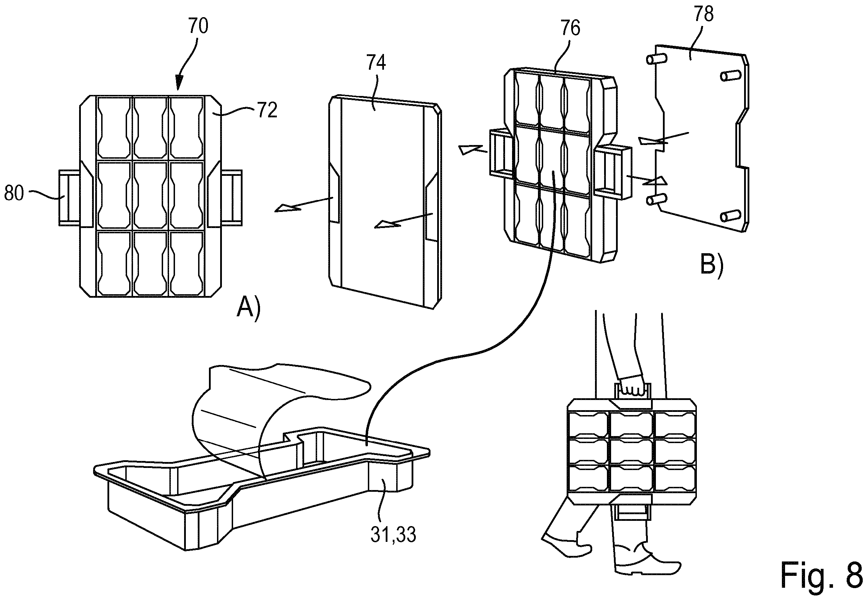

Figur 8 zeigt nun eine alternative Gestaltung eines Dispensers, der hier mit dem Bezugszeichen 70 versehen ist. Der Dispenser 70 enthält ein Gehäuse 72 bestehend aus einem Abdeckelement 74 und einem Rahmenelement 76 sowie einer Rückwand 78, die in einer Explosionsdarstellung in Figur 6B gezeigt sind. Am Rahmenelement sind die in Figur 6A und 6B gezeigten Griffelemente 80 vorgesehen, mittels derer der Dispenser 70 bewegt werden kann. Das Rahmenelement 76 weist Unterteilungen in Form von Fächern auf, wobei in jedem Fach ein Aufnahmekompartment 31, 33 in Form einer Blisterverpackung, die hier knochenförmig ausgebildet ist, eingebracht werden kann. Im Bedarfsfall können dann die einzelnen Aufnahmekompartments 31, 33 aus dem Dispenser 70 entnommen werden.

## Patentansprüche

1. Nothilfematerialeinheit (10), wobei eine Nothilfematerialeinheit (10) eine Zusammenstellung mehrerer Nothilfematerialien, die gemeinsam konfektioniert sind, umfasst, umfassend mindestens ein Aufnahmekompartment (31, 33) zur Aufnahme von Nothilfematerial, wobei jedes Aufnahmekompartment (31, 33) einen geschlossenen, öffenbaren Aufnahmeraum (35) aufweist, der zwischen zwei entlang einer geschlossen verlaufenden Umfangsnaht (37) verbundenen Lagen ausgebildet ist, **dadurch gekennzeichnet, dass** mindestens zwei Aufnahmekompartments (31, 33) vorgesehen sind, wobei jedes der Aufnahmekompartments (31, 33) mindestens ein Griffelement (23) aufweist und wobei das mindestens eine Griffelement (23) bügelförmig (25) ausgebildet ist.

2. Nothilfematerialeinheit nach Anspruch 1, **dadurch gekennzeichnet, dass** mindestens eine, vorzugsweise beide Lagen des Aufnahmekompartments (31, 33) mit einem Griffelement (23) verbunden ist.

3. Nothilfematerialeinheit nach Anspruch 1 oder 2, **dadurch gekennzeichnet, dass** das mindestens eine Griffelement (23) integral mit der jeweiligen Lage außerhalb des Aufnahmeraums (35) verbunden ist.

4. Nothilfematerialeinheit nach einem der vorstehenden Ansprüche, **dadurch gekennzeichnet, dass** sich das mindestens eine Griffelement (23) ausgehend von der Umfangsnaht (37) insbesondere in Flächenrichtung der Lagen erstreckt.

5. Nothilfematerialeinheit nach einem der vorstehenden Ansprüche, **dadurch gekennzeichnet, dass** das mindestens eine Aufnahmekompartment (31, 33) als Blisterverpackung ausgebildet ist und eine der Lagen als Schalenelement (43) und die andere Lage als Deckelelement (45) ausgebildet ist.

6. Nothilfematerialeinheit nach einem der vorstehenden Ansprüche, **dadurch gekennzeichnet, dass** die Lagen aus Kunststofffolie bestehen, insbesondere aus Polyamid, Polypropylen, Polyethylen oder Polyethylenterephthalat.

7. Nothilfematerialeinheit nach einem der vorstehenden Ansprüche, **dadurch gekennzeichnet, dass** die mindestens zwei Aufnahmekompartments (31, 33) miteinander verbunden oder verbindbar sind, vorzugsweise lösbar verbindbar sind.

8. Nothilfematerialeinheit nach einem der vorstehenden Ansprüche, **dadurch gekennzeichnet, dass** die Griffelemente (23) insbesondere einander flächig überdeckend ausgebildet sind.

9. Nothilfematerialeinheit nach einem der vorstehenden Ansprüche, **dadurch gekennzeichnet, dass** die Aufnahmekompartments (31, 33) und/oder die Griffelemente (23) in Richtung senkrecht zur Flächenrichtung übereinander anordenbar sind und sich in dieser Anordnung flächenmäßig überdecken.

10. Nothilfematerialeinheit nach einem der vorstehenden Ansprüche, **dadurch gekennzeichnet, dass** die Aufnahmekompartments (31, 33) untereinander über ein Filmscharnier (41) insbesondere an einer nicht das Griffelement (23) aufweisenden Seitenkante verbunden sind, wobei insbesondere entlang des Filmscharniers (41) Sollbruchstellen, insbesondere in Form von Perforationen vorgesehen sind.

11. Nothilfematerialeinheit nach einem der vorstehenden Ansprüche, **dadurch gekennzeichnet, dass** die Aufnahmekompartments (31, 33) über magnetische komplementäre Elemente miteinander lösbar verbindbar sind.

12. Nothilfematerialeinheit nach einem der vorstehenden Ansprüche, **dadurch gekennzeichnet, dass** mindestens eins der Aufnahmekompartements (31, 33) eine Öffnungshilfe aufweist.

13. Nothilfematerialeinheit nach einem der vorstehenden Ansprüche, **dadurch gekennzeichnet, dass** als Öffnungshilfe das Griffelement (23) dient.

14. Nothilfematerialeinheit nach einem der vorstehenden Ansprüche, **dadurch gekennzeichnet, dass** eine Befestigungsöffnung vorgesehen ist, insbesondere bezüglich des Aufnahmeraums (35) dem Griffelement (23) gegenüberliegend, die außerhalb des Aufnahmeraums liegt, zum Befestigen an einer Halteeinrichtung.

15. Nothilfematerialeinheitdispenser, aufweisend eine Nothilfematerialeinheit (10), ein Gehäuse (17) zur Aufnahme der Nothilfematerialeinheit (10) und eine Gehäuseöffnung (19), durch die die Nothilfematerialeinheit (10) entnommen werden kann, **dadurch gekennzeichnet, dass** die Nothilfematerialeinheit (10) mindestens ein Aufnahmekompartment (31, 33) zur Aufnahme von Nothilfematerial umfasst, wobei jedes Aufnahmekompartment (31, 33) einen geschlossenen, öffenbaren Aufnahmeraum (35) aufweist, der zwischen zwei entlang einer geschlossen verlaufenden Umfangsnaht (37) verbundenen Lagen ausgebildet ist und wobei mit mindestens einem Aufnahmekompartment (31, 33) ein Griffelement (23) verbunden ist und die Nothilfematerialeinheit die Merkmale der Ansprüche 1 bis 14 aufweist.

16. Nothilfematerialeinheitdispenser nach Anspruch 15, **dadurch gekennzeichnet, dass** das Gehäuse (17) und die Gehäuseöffnung (19) so ausgebildet ist, dass sich die Griffelemente (23) bei in das Gehäuse (17) aufgenommener Nothilfematerialeinheit (10) aus der Gehäuseöffnung (15) heraus erstrecken.

17. Nothilfematerialeinheitdispenser nach Anspruch 15 oder 16, **dadurch gekennzeichnet, dass** das Gehäuse (17) eine Rückwand (54) und eine Abdeckung (52) aufweist, die zur Bildung des Gehäuses (17) lösbar miteinander verbindbar sind.

18. Nothilfematerialeinheitdispenser nach einem der vorangehenden Ansprüche 15 bis 17, **dadurch gekennzeichnet, dass** das Gehäuse ein Schutzelement (81) aufweist, das die Gehäuseöffnung (19) verschließt oder verkleinert und wobei das Schutzelement (81) insbesondere verschwenkbar an dem Gehäuse (17) gehalten ist.

19. Nothilfematerialeinheitdispenser nach einem der vorangehenden Ansprüche 15 bis 18, **dadurch gekennzeichnet, dass** der Dispenser (12) im Gehäuse (17) eine Halteeinrichtung (60) aufweist, die eine Befestigungsöffnung (64) in der Nothilfematerialeinheit (10) durchgreift.

20. Nothilfematerialeinheitdispenser nach einem der vorangehenden Ansprüche 15 bis 19, **dadurch gekennzeichnet, dass** der Nothilfematerialeinheitdispenser (12) mit einer Kommunikationseinrichtung, insbesondere einer optischen oder akustischen Signaleinrichtung, einer Kameraeinrichtung und/oder einer Codierung ausgestattet ist.

## Claims

1. Emergency aid material unit (10), an emergency aid material unit (10) comprising a compilation of a plurality of emergency aid materials which are assembled together, the unit comprising at least one accommodating compartment (31, 33) for accommodating emergency aid material, each accommodating compartment (31, 33) having a closed, openable accommodating space (35) which is formed between two layers connected along a closed circumferential seam (37), **characterized in that** at least two accommodating compartments (31, 33) are provided, each of the accommodating compartments (31, 33) having at least one handle element (23) and the at least one handle element (23) being bow-shaped (25).

2. Emergency aid material unit according to claim 1, **characterized in that** at least one, preferably both layers of the accommodating compartment (31, 33) is connected to a handle element (23).

3. Emergency aid material unit according to claim 1 or claim 2, **characterized in that** the at least one handle element (23) is integrally connected to the relevant layer outside the accommodating space (35).

4. Emergency aid material unit according to any of the preceding claims, **characterized in that** the at least one handle element (23) extends from the circumferential seam (37) in particular in the surface direction of the layers.

5. Emergency aid material unit according to any of the preceding claims, **characterized in that** the at least one accommodating compartment (31, 33) is formed as a blister pack, and one of the layers is formed as a shell element (43) and the other layer as a cover element (45).

6. Emergency aid material unit according to any of the preceding claims, **characterized in that** the layers are made of plastics film, in particular polyamide, polypropylene, polyethylene or polyethylene terephthalate.

7. Emergency aid material unit according to any of the preceding claims, **characterized in that** the at least two accommodating compartments (31, 33) are connected or connectable to one another, preferably are detachably connectable.

8. Emergency aid material unit according to any of the preceding claims, **characterized in that** the handle elements (23) are in particular formed to superficially overlap one another.

9. Emergency aid material unit according to any of the preceding claims, **characterized in that** the accommodating compartments (31, 33) and/or the handle elements (23) are arrangeable one above the other in a direction perpendicular to the surface direction and overlap one another in terms of surface area in this arrangement.

10. Emergency aid material unit according to any of the preceding claims, **characterized in that** the accommodating compartments (31, 33) are connected to one another via a film hinge (41), in particular on a lateral edge not having the handle element (23), with predetermined breaking points, in particular in the form of perforations, being provided in particular along the film hinge (41).

11. Emergency aid material unit according to any of the preceding claims, **characterized in that** the accommodating compartments (31, 33) are detachably connectable to one another via magnetic complementary elements.

12. Emergency aid material unit according to any of the preceding claims, **characterized in that** at least one of the accommodating compartments (31, 33) has an opening aid.

13. Emergency aid material unit according to any of the preceding claims, **characterized in that** the handle element (23) serves as an opening aid.

14. Emergency aid material unit according to any of the preceding claims, **characterized in that** a fastening opening is provided, in particular opposite the handle element (23) with respect to the accommodating space (35), which fastening opening lies outside the accommodating space, for fastening to a holding device.

15. Emergency aid material unit dispenser, comprising an emergency aid material unit (10), a housing (17) for accommodating the emergency aid material unit (10) and a housing opening (19) through which housing opening the emergency aid material unit (10) can be removed, **characterized in that** the emergency aid material unit (10) comprises at least one accommodating compartment (31, 33) for accommodating emergency aid material, each accommodating compartment (31, 33) having a closed, openable accommodating space (35) which is formed between two layers connected along a closed circumferential seam (37), a handle element (23) being connected to at least one accommodating compartment (31, 33), and the emergency aid material unit having the features of claims 1 to 14.

16. Emergency aid material unit dispenser according to claim 15, **characterized in that** the housing (17) and the housing opening (19) are formed such that the handle elements (23) extend out of the housing opening (15) when the emergency aid material unit (10) is accommodated in the housing (17).

17. Emergency aid material unit dispenser according to claim 15 or claim 16, **characterized in that** the housing (17) has a rear wall (54) and a cover (52), which are detachably connectable to one another to form the housing (17).

18. Emergency aid material unit dispenser according to any of the preceding claims 15 to 17, **characterized in that** the housing has a protective element (81) which closes or reduces the housing opening (19), the protective element (81) being held in particular pivotably on the housing (17).

19. Emergency aid material unit dispenser according to any of the preceding claims 15 to 18, **characterized in that** the dispenser (12) has a holding device (60) in the housing (17), which holding device passes through a fastening opening (64) in the emergency aid material unit (10).

20. Emergency aid material unit dispenser according to any of the preceding claims 15 to 19, **characterized in that** the emergency aid material unit dispenser (12) is equipped with a communication device, in particular an optical or acoustic signaling device, a camera device and/or a code.

## Revendications

1. Unité de matériel de secours d'urgence (10), dans laquelle une unité de matériel de secours d'urgence (10) comprend un ensemble de plusieurs matériels de secours d'urgence qui sont confectionnés en commun, comprenant au moins un compartiment de réception (31, 33) pour recevoir du matériel de secours d'urgence, dans laquelle chaque compartiment de réception (31, 33) présente un espace de réception (35) fermé et apte à être ouvert qui est réalisé entre deux couches reliées le long d'une couture circonférentielle fermée (37), **caractérisée par le fait qu'**au moins deux compartiments de réception (31, 33) sont prévus, dans laquelle chacun des compartiments de réception (31, 33) présente au moins un élément de poignée (23) et dans laquelle ledit au moins un élément de poignée (23) est réalisé en forme d'étrier (25).

2. Unité de matériel de secours d'urgence selon la revendication 1, **caractérisée par le fait qu'**au moins une, de préférence les deux couches du compartiment de réception (31, 33) est reliée à un élément de poignée (23).

3. Unité de matériel de secours d'urgence selon la revendication 1 ou 2, **caractérisée par le fait que** ledit au moins un élément de poignée (23) est relié d'un seul tenant à la couche respective à l'extérieur de l'espace de réception (35).

4. Unité de matériel de secours d'urgence selon l'une quelconque es revendications précédentes, **caractérisée par le fait que** ledit au moins un élément de poignée (23) s'étend à partir de la couture circonférentielle (37) en particulier dans le sens de la surface des couches.

5. Unité de matériel de secours d'urgence selon l'une quelconque des revendications précédentes, **caractérisée par le fait que** ledit au moins un compartiment de réception (31, 33) est réalisé en tant qu'emballage en blister et qu'une des couches est réalisée en tant qu'élément de coque (43) et l'autre couche est réalisée comme élément de couvercle (45).

6. Unité de matériel de secours d'urgence selon l'une quelconque des revendications précédentes, **caractérisée par le fait que** les couches sont constituées de film plastique, en particulier de polyamide, de polypropylène, de polyéthylène ou de polyéthylène téréphtalate.

7. Unité de matériel de secours d'urgence selon l'une quelconque des revendications précédentes, **caractérisée par le fait que** lesdits au moins deux compartiments de réception (31, 33) sont reliés ou peuvent être reliés l'un à l'autre, de préférence peuvent être reliés de manière amovible.

8. Unité de matériel de secours d'urgence selon l'une quelconque des revendications précédentes, **caractérisée par le fait que** les éléments de poignée (23) sont réalisés en particulier de manière à se chevaucher en surface.

9. Unité de matériel de secours d'urgence selon l'une quelconque des revendications précédentes, **caractérisée par le fait que** les compartiments de réception (31, 33) et/ou les éléments de poignée (23) peuvent être disposés les uns au-dessus des autres dans la direction perpendiculaire à la direction de surface et se chevauchent, dans cette disposition, en termes de surface.

10. Unité de matériel de secours d'urgence selon l'une quelconque des revendications précédentes, **caractérisée par le fait que** les compartiments de réception (31, 33) sont reliés entre eux par l'intermédiaire d'une charnière film (41), en particulier sur un bord latéral ne présentant pas l'élément de poignée (23), dans laquelle des points destinés à la rupture, en particulier sous forme de perforations, sont prévus en particulier le long de la charnière film (41).

11. Unité de matériel de secours d'urgence selon l'une quelconque des revendications précédentes, **caractérisée par le fait que** les compartiments de réception (31, 33) peuvent être reliés entre eux de manière amovible par l'intermédiaire d'éléments magnétiques complémentaires.

12. Unité de matériel de secours d'urgence selon l'une quelconque des revendications précédentes, **caractérisée par le fait que** l'un au moins des compartiments de réception (31, 33) présente une aide à l'ouverture.

13. Unité de matériel de secours d'urgence selon l'une quelconque des revendications précédentes, **caractérisée par le fait que** l'élément de poignée (23) fait fonction d'aide à l'ouverture.

14. Unité de matériel de secours d'urgence selon l'une quelconque des revendications précédentes, **caractérisée par le fait qu'**une ouverture de fixation est prévue, en particulier de manière opposée à l'élément de poignée (23) par rapport à l'espace de réception (35), qui est située à l'extérieur de l'espace de réception, pour la fixation à un dispositif de maintien.

15. Distributeur d'unité de matériel de secours d'urgence, comprenant une unité de matériel de secours d'urgence (10), un boîtier (17) destiné à recevoir l'unité de matériel de secours d'urgence (10) ainsi qu'une ouverture de boîtier (19) à travers laquelle l'unité de matériel de secours d'urgence (10) peut être retirée, **caractérisé par le fait que** l'unité de matériel de secours d'urgence (10) comprend au moins un compartiment de réception (31, 33) destiné à recevoir du matériel de secours d'urgence, dans lequel chaque compartiment de réception (31, 33) présente un espace de réception (35) fermé et apte à être ouvert qui est réalisé entre deux couches reliées le long d'une couture circonférentielle fermée (37), et dans lequel un élément de poignée (23) est relié à au moins un compartiment de réception (31, 33) et l'unité de matériel de secours d'urgence présente les caractéristiques des revendications 1 à 14.

16. Distributeur d'unité de matériel de secours d'urgence selon la revendication 15, **caractérisé par le fait que** le boîtier (17) et l'ouverture de boîtier (19) sont conçus de telle sorte que les éléments de poignée (23) s'étendent hors de l'ouverture de boîtier (15) lorsque l'unité de matériel de secours d'urgence (10) est logé dans le boîtier (17).

17. Distributeur d'unité de matériel de secours d'urgence selon la revendication 15 ou 16, **caractérisé par le fait que** le boîtier (17) présente une paroi arrière (54) et une couverture (52) qui peuvent être reliés entre elles de manière amovible pour former le boîtier (17).

18. Distributeur d'unité de matériel de secours d'urgence selon l'une quelconque des revendications précédentes 15 à 17, **caractérisé par le fait que** le boîtier présente un élément de protection (81) qui ferme ou réduit la taille de l'ouverture de boîtier (19) et dans lequel l'élément de protection (81) est maintenu sur le boîtier (17) en particulier de manière à pouvoir être pivoté.

19. Distributeur d'unité de matériel de secours d'urgence selon l'une quelconque des revendications précédentes 15 à 18, **caractérisé par le fait que** le distributeur (12) présente un dispositif de maintien (60) dans le boîtier (17), qui traverse une ouverture de fixation (64) dans l'unité de matériel de secours d'urgence (10).

20. Distributeur d'unité de matériel de secours d'urgence selon l'une quelconque des revendications précédentes 15 à 19, **caractérisé par le fait que** le distributeur d'unité de matériel de secours d'urgence (12) est équipé d'un dispositif de communication, en particulier d'un dispositif de signalisation optique ou acoustique, d'un dispositif caméra et/ou d'un codage.
